# EUROPEAN PATENT APPLICATION

(11) **EP 4 464 304 A1**
(43) Date of publication of application: **20.11.2024**
(21) Application number: 22920123.1
(22) Date of filing: 14.12.2022
(51) Int. Cl.: A61K 8/02, A61Q 19/10, A61K 8/19, A61K 8/362, A61K 8/23, A61K 8/92

(54) **BATH BOMB**

(30) Priority: 11.01.2022 ES 202230015
(71) Applicant: Válquer Laboratorios, S.L.U., 45440 Toledo Villaminaya (ES)
(72) Inventor: CERRILLO GARCIA-OCHOA, Jose Luis, 45440 VILLAMINAYA (TOLEDO) (ES)
(74) Representative: Cueto, Sénida
(86) International application number: PCT/ES2022/070802
(87) International publication number: WO 2023/135347

(57) **Abstract**

Disclosed is a bath bomb comprising: 45-65% first buffer (AM1); at least 20-30% second buffer (AM2); 2-8% first conditioner (AC1); 4-10% second conditioner (AC2); 0.1-0.6% emulsifying surfactant (S); 1-4% perfume (P); and 0.1-0.5% solvent (D). Also disclosed is a method for producing a bath bomb, which comprises: preparing (1) ingredients by weighing them and crushing (1.2) the first conditioner (AC1); mixing (4) all the ingredients except the second buffer (AM2); checking (5) the mixture; crushing (6) the second buffer (AM2); adding (7) the crushed second buffer to a mixer and stirring; carrying out manual stirring (8); and stirring (9) in the mixer.

## Description

### Technical field of the invention

The present invention corresponds to the technical field of relaxation and personal care products, such as gels, bath salts and, in particular, to a bath bomb.

### Background of the Invention

Nowadays, with the fast pace of life, it is necessary to find moments of disconnection in which to forget about the cell phone, work, pending tasks and obligations. These moments can be found in a coffee with friends, a while of reading, or a relaxing bath.

The bath is an economical way to disconnect and relax, and there are many products that help to create pleasant conditions for that moment. Some of them are the traditional bath salts, candles, relaxing music... In addition, a product known as bath bombs, which has a great acceptance, has been a trend for some time now.

Bath bombs are a product that releases exquisite aromas, oils, extracts and natural butters into the bath water, which provide hydration and softness to the skin and generate a fun and special experience for the bathing ritual, thanks to its colorful and effervescence, and also manage to revitalize and relax the mind and body.

In some cases, these bath bombs can have a relaxing and anti-inflammatory effect and, in other cases where the opposite effect is desired, with the right ingredients they can be revitalizing and provide the user with an energy recharge.

Bath bombs, when placed in water and starting their effervescence, provide an intense fragrance, lightly color the water and majestically fill the bathtub with foam.

Although they are highly demanded products, they really have some aspects to improve, such as the fact that their effect is limited to something merely decorative both visually, as they color the water, generate a lot of foam, and some have the effect of spinning on themselves, and olfactory, as they scent the water noticeably and this aroma persists on the user's skin to a greater or lesser extent.

However, it would be desirable for them to simultaneously provide deep hydration to the user's skin, but this does not happen. Although some of these bath bombs claim to contain butters and essential oils among their ingredients, aiming for the desired hydration, the levels of hydration achieved are minimal since the amounts of these ingredients must be reduced to avoid a greasy appearance of the bath bombs.

### Description of the invention

The bath bomb disclosed herein comprises a first buffering agent formed by sodium bicarbonate, in a percentage comprised between 45 and 65% and at least a second buffering agent formed by citric acid, in a percentage comprised between 20 and 30%.

Furthermore, this bath bomb comprises a first conditioning agent formed by magnesium sulfate, in a percentage between 2 and 8%, and a second conditioning agent formed by is *prunus amygdalus dulcis* oil (sweet almond oil), in a percentage between 4 and 10%.

It also contains a surfactant-emulsifying agent, in a percentage between 0.1 and 0.6%, a fragance, in a percentage between 1 and 4%, and a solvent which is water, in a percentage between 0.1 and 0.5.

This document also proposes a method for manufacturing a bath bomb, as previously defined comprising a first stage of preparation of a first set of ingredients formed by a first buffering agent, a first conditioning agent and a surfactant-emulsifying agent.

In this first phase, each ingredient is weighed and the first conditioning agent is sprayed until a powdered texture is achieved.

A second phase is then carried out, involving the preparation of a second set of ingredients formed by a second conditioning agent, a perfume and a solvent. In this second phase, each ingredient is weighed and all of them are mixed and stirred in the same container.

A third phase consists of preparing at least one second buffering agent, in which it is weighed.

Then, a fourth phase is formed by a kneading of the first and second set of ingredients in a mixer, with a stirring speed of 30 rpm for 70 sec.

At the end of this phase, a fifth phase verifies the homogeneity of the mixture obtained from the kneading.

A sixth step consists of pulverizing the second buffering agent to a powder texture, followed by a seventh step of adding at least the second buffering agent to the mixer and stirring at a speed of 20 rpm for 1 minute.

The process is completed with an eighth phase of additional manual stirring by means of a paddle, and a ninth phase consisting of a new stirring in the mixer at a speed of 30 rpm for 40 seconds.

With the bath bomb and the manufacturing process proposed here, a significant improvement over the state of the art is achieved.

This is because a bath bomb with a high content of vegetable oil, such as sweet almond oil, is obtained, providing skin hydration levels of around 30%.

This is possible thanks to the balance achieved between the other ingredients and the sweet almond oil, as well as obtaining the appropriate particle size of the solid ingredients, eliminating the greasy feel while providing the appropriate consistency and hardness of these bath bombs.

### Brief description of the drawings

In order to help a better understanding of the characteristics of the invention, in accordance with a preferred example of practical realization thereof, it is provided as an integral part of said description, a series of drawings where, with illustrative and not limiting character, the following has been represented:

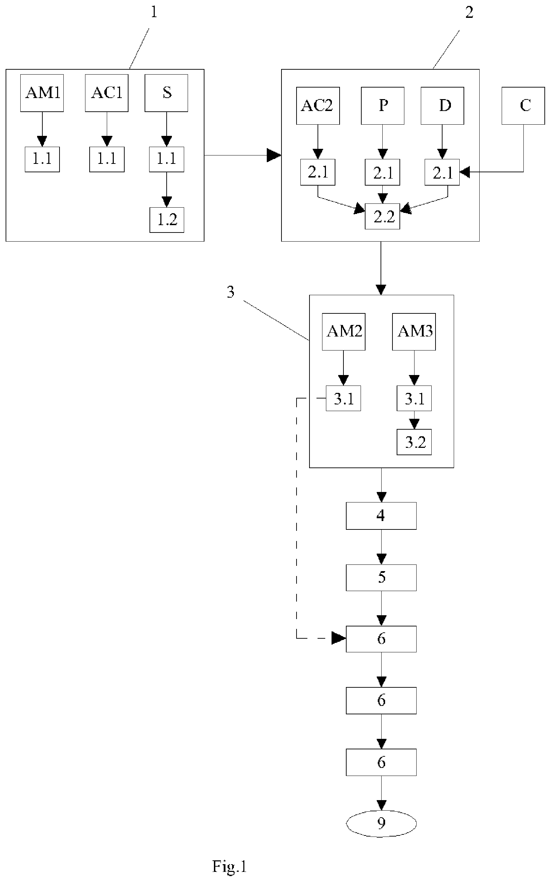

Figure 1.- Shows a block diagram of the procedure for making a bath bomb, for a first preferred embodiment of the invention.

### Detailed description of a preferred embodiment of the invention

In a preferred embodiment of the invention, the bath bomb proposed herein comprises a first buffering agent (AM1) consisting of sodium bicarbonate, in a percentage comprised between 45 and 65% and preferably between 50 and 60%.

It also comprises at least a second buffering agent (AM2) consisting of citric acid, in a percentage of between 20 and 30%, which in this embodiment is preferably between 21 and 25%.

This bath bomb comprises a first conditioning agent (AC1) formed by magnesium sulfate, in a percentage between 2 and 8% and a second conditioning agent (AC2) consisting of *prunus amygdalus dulcis* oil, known as sweet almond oil, in a percentage between 4 and 10%. In this preferred embodiment of the invention, the percentage of magnesium sulfate is preferably between 4 and 6% and the percentage of sweet almond oil between 5 and 8%, preferably.

It also presents a surfactant-emulsifying agent (S), in a percentage comprised between 0.1 and 0.6%. In this preferred embodiment of the invention, said surfactant-emulsifying agent (S) is formed by sodium coco-sulfate, being in this case in a preferred form, in a percentage comprised between 0.2 and 0.4%.

Finally, it has a perfume (P), in a percentage between 1 and 4%, and a solvent (D) formed by water, in a percentage between 0.1 and 0.5%.

In this preferred embodiment of the invention, the bath bomb presented herein further comprises a third buffering agent (AM3) consisting of tartaric acid, in a percentage comprised between 2 and 8%.

On the other hand, in this preferred embodiment, the bath bomb comprises one or more dyes (C) in a percentage comprised between 0.01 and 0.1%. In this particular case it comprises a colorant consisting of CI 17200.

This report also proposes a process for the production of a bath bomb, as defined above, and this process has a series of stages which are described below.

Thus, as shown in Figure 1, the procedure presents a first preparation phase (1) of a first set of ingredients formed by a first buffering agent (AM1), a first conditioning agent (AC1) and a surfactant-emulsifying agent (S). That is to say, in this case the preparation (1) of sodium bicarbonate, magnesium sulfate and sodium coco-sulfate is carried out.

In this first phase, each ingredient is weighed (1.1) and the first conditioning agent (AC1) is sprayed (1.2) until it reaches a powder texture. This ingredient, i.e. magnesium sulfate, can be pulverized prior to use since it does not clump as it rests.

The second phase consists of a preparation (2) of a second set of ingredients formed by a second conditioning agent (AC2), a perfume (P) and a solvent (D), where the weighing (2.1) of each ingredient and the mixing and stirring (2.2) of all of them in the same container is carried out. In this case, these ingredients consist of *prunus amygdalus dulcis* oil, a perfume and a solvent, in this case, water.

In this preferred embodiment, the bath bomb comprises one or more dyes (C), in this case only the dye CI 17200 and the previous preparation step (2) of the second set of ingredients further comprises, the weighing (2.1) of said dye (C) dissolved in the solvent (D).

As can be seen in Figure 1, the procedure presents a third phase consisting of a preparation (3) of at least a second buffering agent (AM2), formed in this case by citric acid, where the weighing (3.1) of the same is performed.

In this preferred embodiment of the invention, the bath bomb further comprises a third buffering agent (AM3), which in this case is formed by tartaric acid, and this third preparation phase (3) therefore consists of weighing (3.1) and independently of both second and third buffering agents (AM2, AM3).

Since there is a third buffering agent (AM3), the third preparation step further comprises spraying (3.2) of the same, until a powder texture is reached and, this spraying (3.2) takes place after weighing (3.1) of the second and third buffering agents (AM2, AM3).

Next, a fourth phase of kneading (4) of the first and second set of ingredients is carried out in a mixer, with a stirring speed of 30 rpm, for 70 s and then a fifth phase of checking (5) of the homogeneity of the mixture obtained in said kneading.

The sixth stage consists of pulverizing (6) the second buffering agent (AM2), i.e. citric acid, to a powdered texture. This ingredient cannot be sprayed at the same time as the tartaric acid, as it must be sprayed at this time, just before use, to avoid possible crystallization.

This is followed by the seventh step of addition (7) of the second and third buffering agents (AM2, AM3) to the mixer and stirring at a speed of 20 rpm for 1 minute.

An eighth phase consists of an additional manual stirring (8) by means of a paddle, and finally, a ninth stirring phase (9) is carried out in the mixer at a speed of 30 rpm for 40 seconds.

## Claims

1. Bath bomb, comprising
- a first buffering agent (AM1) formed by sodium bicarbonate, in a percentage between 45 and 65%;
- at least a second buffering agent (AM2) formed by citric acid, in a percentage between 20 and 30%;
- a first conditioning agent (AC1) formed by magnesium sulfate, in a percentage between 2 and 8%;
- a second conditioning agent (AC2) consisting of *prunus amygdalus dulcis* oil (sweet almond oil), in a percentage between 4 and 10%;
- a surfactant-emulsifying agent (S) in a percentage between 0.1 and 0.6%;
- a perfume (P), in a percentage between 1 and 4%, and;
- a solvent (D) formed by water, in a percentage between 0.1 and 0.5%.

2. Bath bomb, according to claim 1, comprising a third buffering agent (AM3) formed by tartaric acid, in a percentage between 2 and 8%;

3. Bath bomb, according to any of the preceding claims, wherein the surfactant-emulsifying agent (S) is formed by sodium coco-sulfate.

4. Bath bomb, according to any of the preceding claims, comprising one or more dyes (C) in a percentage between 0.01 and 0.1%.

5. Bath bomb, according to claim 5, wherein the dye (C) is formed by CI 17200.

6. Procedure for making a bath bomb, as the one defined in claims 1 to 5, comprising
- a preparation (1) of a first set of ingredients formed by a first buffering agent (AM1), a first conditioning agent (AC1) and a surfactant-emulsifying agent (S), wherein weighing (1.1) of each ingredient and spraying (1.2) of the first conditioning agent (AC1) to a powder texture is performed;
- a preparation (2) of a second set of ingredients formed by a second conditioning agent (AC2), a perfume (P) and, a solvent (D), wherein the weighing (2.1) of each ingredient and the mixing and stirring (2.2) of all of them in the same container is carried out;
- a preparation (3) of at least a second buffering agent (AM2), wherein the weighing (3.1) is carried out;
- a kneading (4) of the first and second set of ingredients in a mixer, with a stirring speed of 30 rpm, for 70 s;
- checking (5) and homogeneity of the mixture obtained in the kneading;
- a spraying (6) of the second buffering agent (AM2), until a powder texture is reached;
- addition (7) of at least a second buffering agent (AM2) to the mixer and stirring at a speed of 20 rpm, for 1 minute;
- supplementary manual stirring (8) by means of a paddle, and;
- stirring (9) in the mixer at a speed of 30 rpm, for 40 s.

7. Procedure for making a bath bomb, according to claim 6, wherein the bath bomb comprises a third buffering agent (AM3) and the preparation phase (3) of at least a second buffering agent comprises the weighing (3.1) of both the second and third buffering agents (AM2, AM3) independently of each other.

8. Procedure for making a bath bomb, according to claim 7, wherein the preparation phase (3) of at least a second buffering agent (AM2) comprises spraying (3.2) of the third buffering agent (AM3), until a powder texture is reached, after weighing (3.1) of the second and third buffering agents (AM2, AM3).

9. Procedure for making a bath bomb, according to any of claims 6 to 8, wherein the bath bomb comprises one or more dyes (C) and the preparation (2) of the second set of ingredients comprises weighing (2.1) said dye dissolved in the solvent.
